# EUROPEAN PATENT APPLICATION

(11) **EP 3 382 393 A1**
(43) Date of publication of application: **03.10.2018**
(21) Application number: 18163084.9
(22) Date of filing: 21.03.2018
(51) Int. Cl.: G01N 33/53

(54) **DEVICE AND METHOD TO SELECT AN ANTIBODY COCKTAIL REAGENT**

(30) Priority: 28.03.2017 JP 2017062559
(71) Applicant: SYSMEX CORPORATION, Kobe-shi Hyogo 651-0073 (JP)
(72) Inventor: Yao, Syunsuke, Kobe-shi Hyogo 651-0073 (JP); Tsuji, Tomohiro, Kobe-shi Hyogo 651-0073 (JP)
(74) Representative: Hoffmann Eitle

(57) **Abstract**

Selecting an appropriate combination of antibody reagents according to a measurement order, without being an examiner who has expert knowledge is made possible.

The reagent selection support apparatus 100 supports the selection of a cocktail antibody reagent containing a plurality of antibodies, and includes acquisition units 15 and 16 that acquire a measurement order with a plurality of antigens as measurement targets, a processing unit 10 for determining a plurality of cocktail antibody reagents to be used for a plurality of assays for detecting a plurality of antigens, based on the measurement order acquired by the acquisition units 15 and 16, and an output unit 17 for outputting information of a plurality of cocktail antibody reagents used for a plurality of assays, the information being determined by the processing unit 10.

## Description

### CROSS REFERENCE TO RELATED APPLICATIONS

This application claims priority from prior Japanese Patent Application No. 2011-085587, filed on March 28, 2017, entitled "INSTRUMENT SETUP SYSTEM FOR FLUORESCENCE ANALYZER",the entire contents of which are incorporated herein by reference.

### FIELD OF THE INVENTION

The present invention relates to a reagent selection support apparatus, a method, a program, a recording medium, and a sample measurement apparatus.

### BACKGROUND

Flow cytometry can be used to detect various antigens present on a cell surface or intracellularly by combining with fluorescent immunostaining as disclosed in, for example, Japanese Patent Application Publication No. 2011-085587 below, which is particularly useful for analysis of cells.

Multicolor flow cytometry capable of detecting multiple antigens in one assay is useful as the number of antigens to be detected increases and the number of assays per sample also increases when multiple kinds of antigens are detected at one time using flow cytometry. In multicolor flow cytometry, it is necessary to preliminarily fluorescently stain an antigen to be detected. For the fluorescent staining of the antigen, a test reagent called a cocktail antibody reagent is used. For the cocktail antibody reagent, a plurality of antibodies that are frequently used are combined in advance after cross-reactivity and the like have been prepared.

However, it is necessary to know in advance that the detection antibody will not show cross-reactivity with antigens simultaneously detected other than the target antigen in order to detect many kinds of antigens in a single assay, and of course antigens are selected according to the type of cells to be detected. If cross-reactivity is observed, information of the characteristics of the antibody is required before applying the immunostained cells to the flow cytometer, such as using different assays for cross-reactive antigens. Therefore, currently, in order to obtain reliable results with high reproducibility in multicolor flow cytometry, it is necessary for the operator who performs multicolor flow cytometry to collect all kinds of antibodies used for flow cytometry, and to know the cross reactivity in advance. Furthermore, since the number of fluorochromes that can be used differs depending on the number of light sources mounted on the flow cytometer to be used and the number of dichroic mirrors, knowledge about the flow cytometer and knowledge about the fluorochromes are also required by the operator. Against this background, current multicolor flow cytometry is a technique that can only be performed by experts with advanced expert knowledge.

On the other hand, flow cytometry is a technology which greatly contributes to diagnosis of disease, determination of the stage of disease, and determination of therapeutic policy. Due to the popularization of flow cytometry, it has become possible to accurately know the cell line of tumor cells, and the diagnosis of hematopoietic tumors heretofore based on cell morphological observation, enzyme staining, and immunostaining has largely migrated to diagnosis based on profiling of cell surface markers. A physician who diagnoses disease relies on the operator when a plurality of kinds of diagnostic antigens are used as measurement items in flow cytometry.

When determining the cell lineage, it is generally necessary to detect 10 to 30 surface markers per sample by simply identifying whether it is, for example, a B cell line tumor or not. In order to detect all targeted surface markers, multicolor flow cytometry using several detection antibodies per assay must be performed multiple times for each sample. In addition, the operator of the flow cytometer must select a combination of suitable detection antibodies corresponding to the measurement items requested by the physician or technician, that is, a combination of detection antibodies from the detection antibodies on hand at the time of the examination. Since the cells used for flow cytometry must be subjected to measurement within 24 hours after collection, it also is necessary to consider the combination of detection antibodies capable of efficiently performing measurement per sample. Under such circumstances, it is necessary to efficiently select a cocktail antibody reagent to be used for fluorescent staining of the detection antibody from the stock of the cocktail antibody reagents on hand.

From the present situation, flow cytometry currently is performed exclusively by an operator possessing knowledge of the characteristics of the detection antibodies, who selects a cocktail antibody reagent according to measurement items requested by a physician or a technician, and the fact is that the operator who has such expert knowledge is occupied full time.

### SUMMARY OF THE INVENTION

There is a need to "provide stable examination results at any time, quickly, whenever necessary" on site at hospital laboratories. Regarding tests using flow cytometry, however, examinations using flow cytometry become ever more complex and greatly diverge from this need as the expertise of the specialist examiner performing flow cytometry increases. Even if a person other than the full-time examiner receives measurement items of plural kinds of antigens from a physician or technician, it is extremely difficult to efficiently select a combination of cocktail antibody reagents for detecting plural kinds of antigens shown in measurement items. Although it is conceivable to detect a plurality of types of antigens contained in measurement items by flow cytometry one by one as described above, this is not realistic since the cells must be subjected to measurement within 24 hours after collection. There is a need for a method of efficiently selecting a combination of cocktail antibody reagents according to measurement items of plural kinds of antigens requested by a physician at the site of examination in hospitals.

Although the method of Japanese Patent Application Publication No. 2011-085587 is a method aimed at simplifying setup of equipment such as a flow cytometer, it is a method premised on the operator having a certain degree of expert knowledge, and is not a user-friendly method targeted at neophytes. The method of Japanese Patent Application Publication No. 2011-085587 does not satisfy the above-mentioned needs which are required for on-site examination at hospitals in the first place.

One embodiment of the invention is a reagent selection support apparatus. In the embodiment, a reagent selection support apparatus (100) supports the selection of a cocktail antibody reagent containing a plurality of antibodies, and includes acquisition units (15 and 16) that acquire a measurement order with a plurality of antigens as measurement targets, processing units (10A and 10B) for determining a plurality of cocktail antibody reagents to be used for a plurality of assays for detecting a plurality of antigens based on the measurement order acquired by the acquisition units (15 and 16), and an output unit (17) for outputting information of a plurality of cocktail antibody reagents used for a plurality of assays, the information being determined by the processing unit. In this way it becomes possible to select an appropriate combination of antibody reagents according to a measurement order, without being an examiner who has expert knowledge.

It is desirable to further provide a storage unit (12, 13) for storing information of a plurality of cocktail antibody reagents composed of combinations of different antibodies, wherein the processing unit (15, 16) determines a plurality of cocktail antibody reagents to be used for multiple assays based on the measurement order and the information of the cocktail antibody reagent. In this way, it is possible to select an appropriate combination of antibody reagents according to the measurement order even if the examiner does not possess expert knowledge based on the information of a plurality of cocktail antibody reagents previously stored in the storage unit.

Each of the plurality of cocktail antibody reagents preferably is composed of a combination of different antibodies. As a result, it is possible to increase the kind of fluorescence that can be measured simultaneously, and it is possible to decrease the number of measurements and reduce the measurement time.

It is desirable that the processing unit (10A, 10B) determines an antibody reagent that can be used in combination with the cocktail antibody reagent in at least one of the plurality of assays, and the output unit (17) outputs the information of the antibody reagent that can be used in combination with the cocktail antibody reagent. As a result, it is possible to increase the kind of fluorescence that can be measured simultaneously, and it is possible to decrease the number of measurements and reduce the measurement time.

Based on the measurement order, t is preferable that the processing unit (10A, 10B) calculates a first candidate to be a combination of cocktail antibody reagents used in a plurality of assays and a second candidate to be a combination of cocktail antibody reagents different from the first candidate, and the output unit (17) outputs the first candidate and the second candidate determined by the processing unit (10A, 10B). As a result, the user can perform an efficient examination using the antibody reagent stocked at hand.

The output unit (17) desirably outputs the first candidate and the second candidate so as to be mutually distinguishable from each other. In this way, the user can more reliably grasp whether a measurement satisfying the measurement order is possible using the antibody reagents stocked at hand.

When the processing unit (10A, 10B) accepts an input designation of the cocktail antibody reagent to be used for either the first candidate or the second candidate, it is preferable that the processing unit (10A, 10B) changes the display mode of the cocktail antibody reagent corresponding to the designation of each of the first candidate and the second candidate. When the user inputs the information of the antibody reagent stocked at hand into the reagent selection support apparatus (100), the display mode of the antibody reagent stocked at hand is changed and output to the output unit (17). In this way, the user can more reliably grasp whether a measurement satisfying the measurement order is possible using the antibody reagents stocked at hand.

It is preferable that the processing unit (10A, 10B) presents the first candidate and the second candidate according to the number of uses of the cocktail antibody reagent and/or the magnitude relationship of the number of measurements. As a result, the user can perform an efficient examination using the antibody reagent stocked at hand.

When there are a plurality of results having the same number of uses of cocktail antibody reagents, it is desirable to present the first candidate and the second candidate according to the magnitude relationship of the number of measurements among the first candidate and the second candidate having the same number of uses the cocktail antibody reagent. As a result, the user can perform an efficient examination using the antibody reagent stocked at hand.

It is preferable that the processing unit (10A, 10B) presents the first candidate and the second candidate according to the extent to which the plurality of antibodies contained in the cocktail antibody reagent matches the plurality of antigens. As a result, the user can perform an efficient examination using the antibody reagent stocked at hand.

One embodiment of the present invention is a reagent selection support method. In an embodiment, the reagent selection support method is a reagent selection support method for supporting selection of a cocktail antibody reagent containing a plurality of antibodies, the method including a step (S1) of acquiring a measurement order having a plurality of antigens as measurement targets, a step (S2 to S5) of determining a plurality of cocktail antibody reagents to be used in a plurality of assays for detecting a plurality of antigens based on the obtained measurement order, a step (S6) of outputting information of the plurality of cocktail antibody reagents to be used in the determined plurality of assays. In this way it becomes possible to select an appropriate combination of antibody reagents according to a measurement order, without being an examiner who has expert knowledge.

It is desirable to further include a step of reading out information of a plurality of cocktail antibody reagents configured by combinations of different antibodies from a storage unit, and the determining step reads out the information stored in the storage unit in the reading step and determines multiple cocktail antibody reagents to use for multiple assays based on the measurement order and information of the multiple cocktail antibody reagents. In this way, it is possible to select an appropriate combination of antibody reagents according to the measurement order even if the examiner does not possess expert knowledge based on the information of a plurality of cocktail antibody reagents previously stored in the storage unit.

Each of the plurality of cocktail antibody reagents preferably is composed of a combination of different antibodies. As a result, it is possible to increase the kind of fluorescence that can be measured simultaneously, and it is possible to decrease the number of measurements and reduce the measurement time.

It is desirable that the determining step determines an antibody reagent that can be used in combination with the cocktail antibody reagent in at least one of the plurality of assays, and the outputting step outputs the information of the antibody reagent that can be used in combination with the cocktail antibody reagent. As a result, it is possible to increase the kind of fluorescence that can be measured simultaneously, and it is possible to decrease the number of measurements and reduce the measurement time.

It is desirable that the determining step determines a first candidate to be a combination of a cocktail antibody reagent to be used for a plurality of assays and a second candidate to be a combination cocktail antibody reagent different from the first candidate based on a measurement order, and the outputting step outputs the first candidate and the second candidate determined in the determining step. As a result, the user can perform an efficient examination using the antibody reagent stocked at hand.

It is desirable that the outputting step outputs the first candidate and the second candidate so as to be mutually distinguishable from each other. In this way, the user can more reliably grasp whether a measurement satisfying the measurement order is possible using the antibody reagents stocked at hand.

It is desirable to further include a step of accepting an input designation of a cocktail antibody reagent to be used in either one of the first candidate and the second candidate, and a step of changing the display mode of the cocktail antibody reagent corresponding to the designation of each of the first candidate and the second candidate. When the user inputs the information of the antibody reagent stocked at hand into the reagent selection support apparatus (100), the display mode of the antibody reagent stocked at hand is changed and output to the output unit (17). In this way, the user can more reliably grasp whether a measurement satisfying the measurement order is possible using the antibody reagents stocked at hand.

The determining step preferably presents the first candidate and the second candidate according to the number of uses the cocktail antibody reagent and/or the magnitude relationship of the number of measurements. As a result, the user can perform an efficient examination using the antibody reagent stocked at hand.

It is preferable that the determining step presents the first candidate and the second candidate according to the magnitude relationship of the number of measurements among the first candidate and the second candidate having the same number of uses of the cocktail antibody reagent when there are a plurality of results having the same number of uses of cocktail antibody reagents. As a result, the user can perform an efficient examination using the antibody reagent stocked at hand.

The determining step preferably presents the first candidate and the second candidate according to the extent to which a plurality of antibodies contained in the cocktail antibody reagent match a plurality of antigens. As a result, the user can perform an efficient examination using the antibody reagent stocked at hand.

One embodiment of the invention is a computer program. In an embodiment, a computer program causes a computer to realize an acquisition function (S1) for acquiring a measurement order for measuring a plurality of antigens, a determination function (S2 to S5) for determining a plurality of cocktail antibody reagents to be used in a plurality of assays for detecting a plurality of antigens based on the obtained measurement order, and an output function (S6) for outputting information of a plurality of cocktail antibody reagents used for the determined plurality of assays.

One embodiment of the present invention is a computer readable non-transitory tangible recording medium. In an embodiment, a computer readable, non-transitory tangible recording medium on which is recorded a program to realize an acquisition function (S1) for acquiring a measurement order having a plurality of antigens as measurement targets, a determination function (S2 to S5) for determining a plurality of cocktail antibody reagents to be used in a plurality of assays for detecting a plurality of antigens based on the obtained measurement order, and an output function (S6) for outputting information of a plurality of cocktail antibody reagents used for the determined plurality of assays.

One embodiment of the invention is a reagent selection support apparatus. In the embodiment, a reagent selection support apparatus (100) supports the selection of a cocktail antibody reagent containing a plurality of antibodies, and includes acquisition units (15 and 16) that acquire a measurement order designating a plurality of antigens as measurement targets, processing units (10A and 10B) for determining a plurality of cocktail antibody reagents to be used for a plurality of assays for detecting a plurality of antigens based on the measurement order acquired by the acquisition units (15 and 16), and an output unit (17) for outputting information of a plurality of cocktail antibody reagents used for a plurality of assays, the information being determined by the processing unit.

One embodiment of the present invention is a sample measurement apparatus. In an embodiment, a sample measuring apparatus (1000) for measuring a sample (29) in which a cocktail antibody reagent containing a plurality of antibodies and a plurality of antigens to be measured are mixed, the apparatus includes a reagent selection support device (100) for determining a plurality of cocktail antibody reagents to be used in a plurality of assays for detecting a plurality of antigens in sample (29), and a measurement apparatus body (200) including a suction unit (21) for suctioning a sample (29) in which a plurality of cocktail antibody reagents used in a plurality of assays are mixed for each assay, a fluid circuit (22) for transporting the sample (29) suctioned by the suction unit (21), and a detection unit (23) for measuring the sample (29) carried by the fluid circuit (22).

According to the present invention, it is possible to select an appropriate combination of antibody reagents according to the measurement order, without being an examiner who has expert knowledge.

### BRIEF DESCRIPTION OF THE DRAWINGS

FIG. 1 is an example of a screen display of a reagent selection support apparatus;
FIG. 2 is a schematic diagram describing an examination system according to a first embodiment;

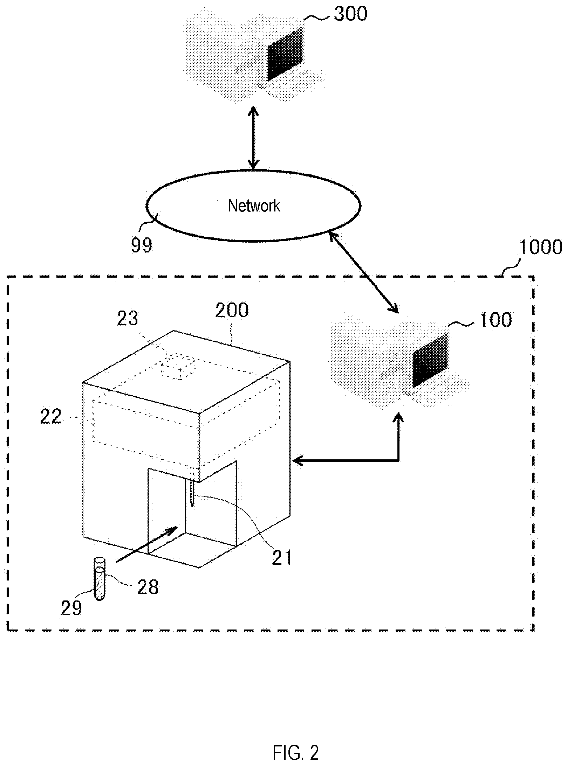

FIG. 2 is a brief diagram showing an optical system of a flow cytometer;

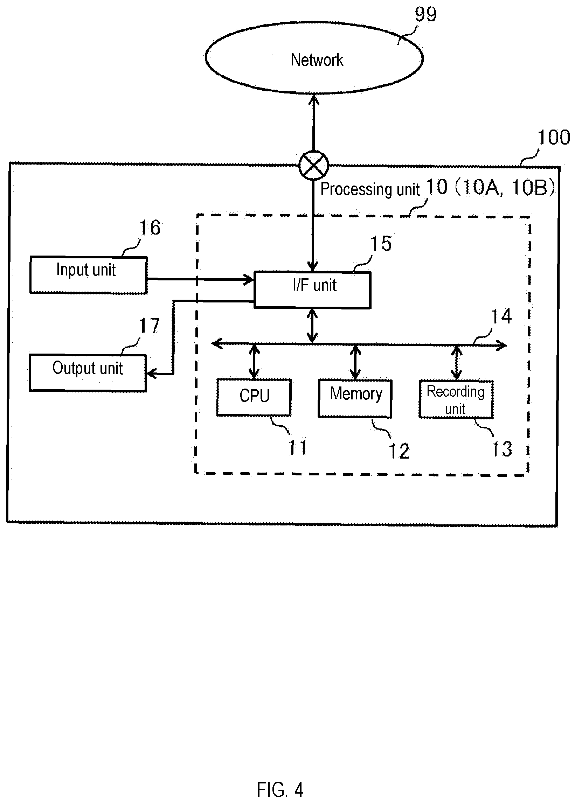

FIG. 4 is a block diagram showing a hardware configuration of a reagent selection support apparatus;

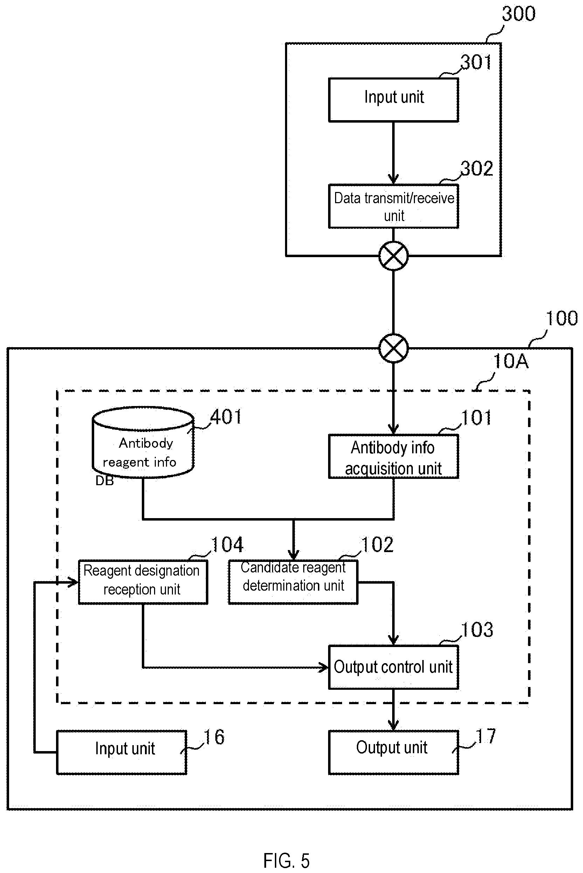

FIG. 5 is a block diagram explaining the functions of the examination system according to the first embodiment;

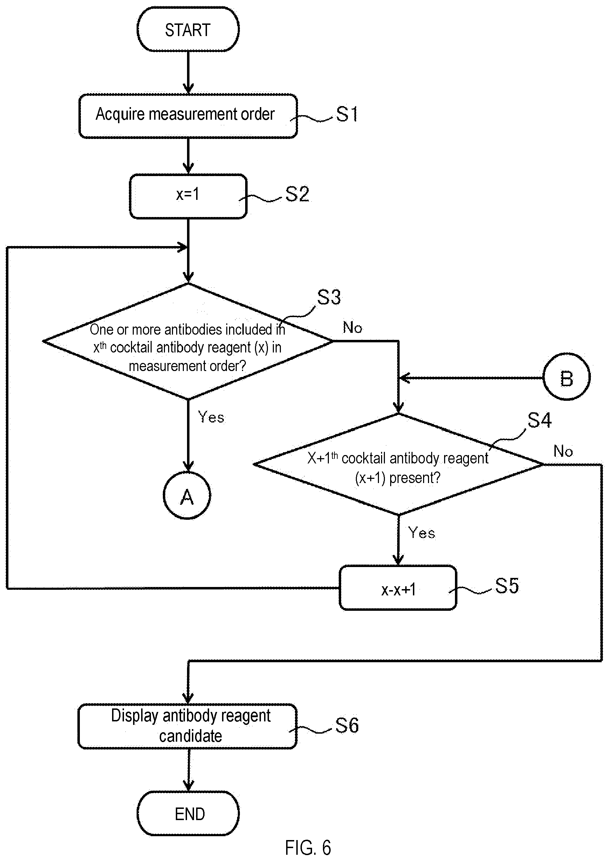

FIG. 6 is a flowchart showing a procedure for determining candidates for a combination of antibody reagents;

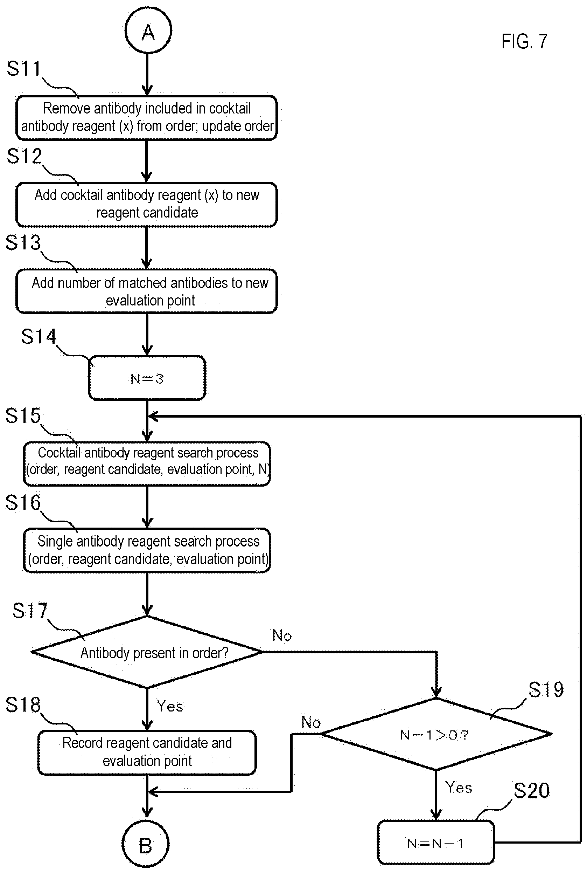

FIG. 7 is a flowchart showing a procedure for determining candidates for a combination of antibody reagents;

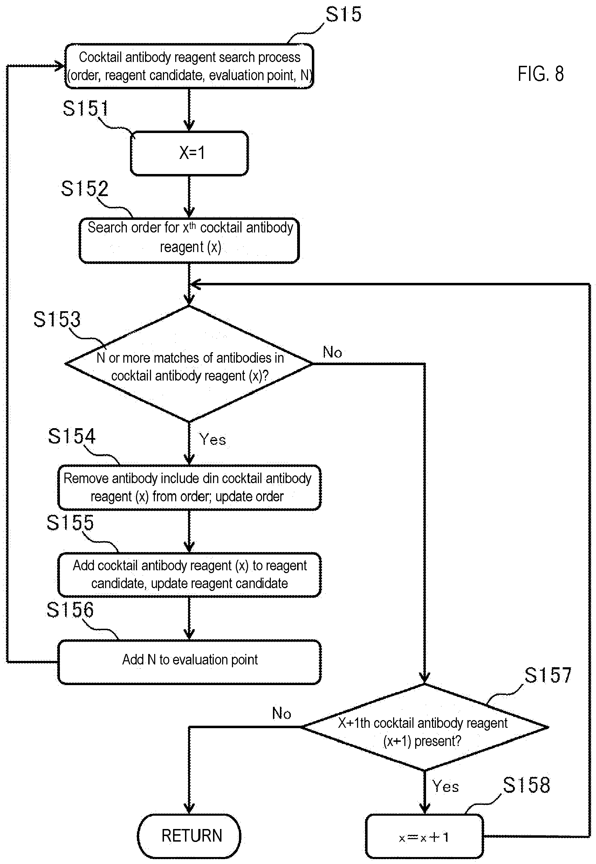

FIG. 8 is a flowchart showing a procedure for determining candidates for a combination of antibody reagents;

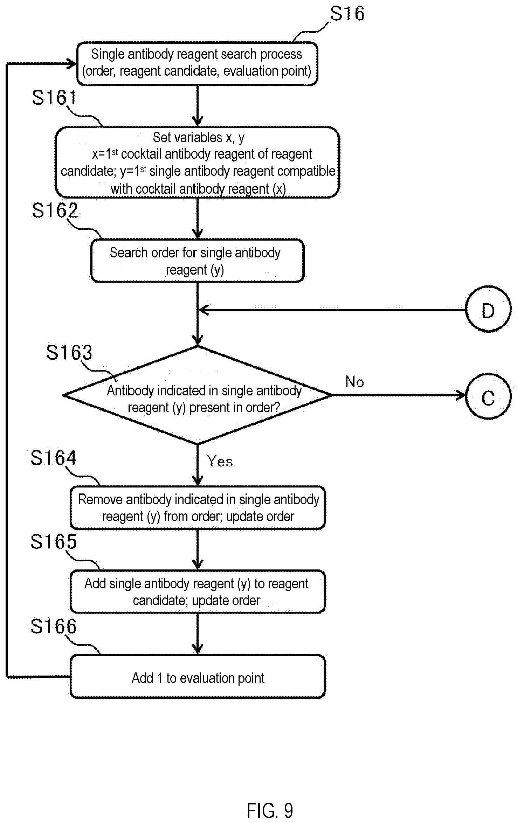

FIG. 9 is a flowchart showing a procedure for determining candidates for a combination of antibody reagents;

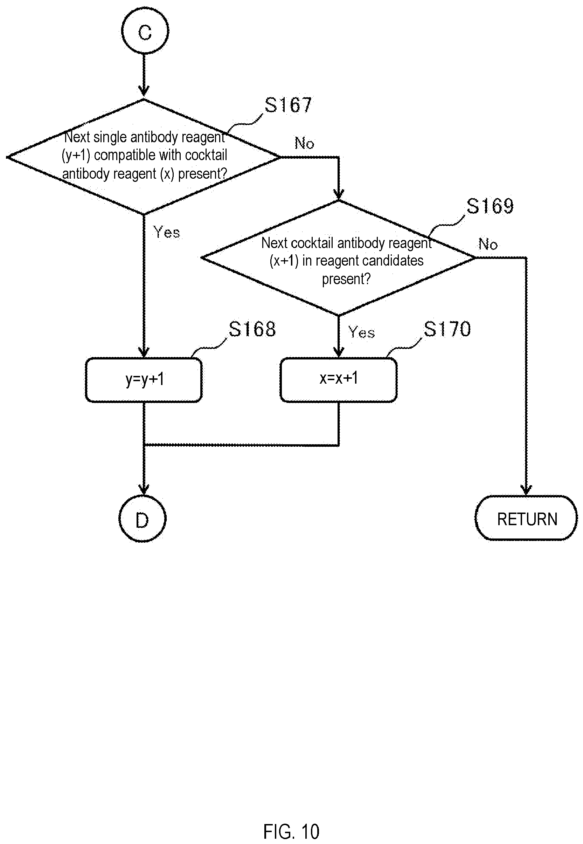

FIG. 10 is a flowchart showing a procedure for determining candidates for a combination of antibody reagents;

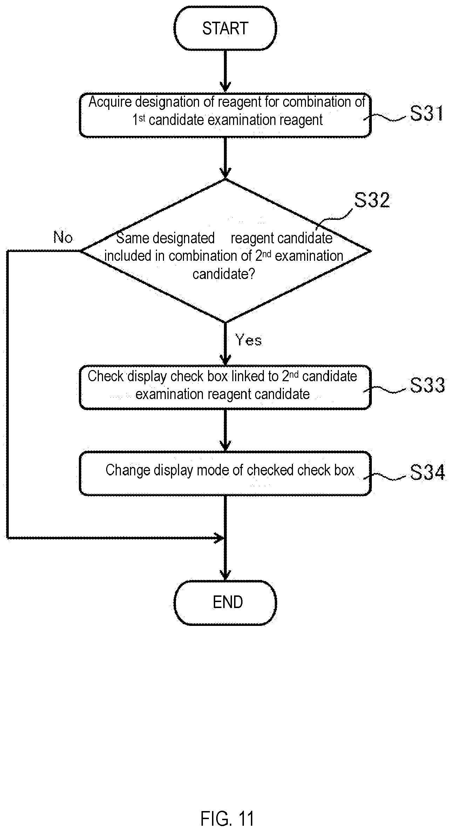

FIG. 11 is a flowchart showing a procedure of comprehending a combination of antibody reagents satisfying a measurement order;

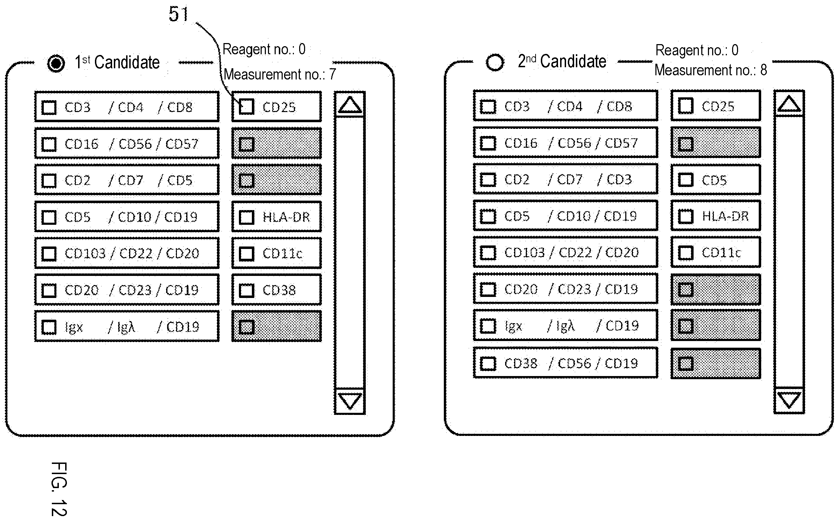

FIG. 12 is an example of screen display of the reagent selection support apparatus;

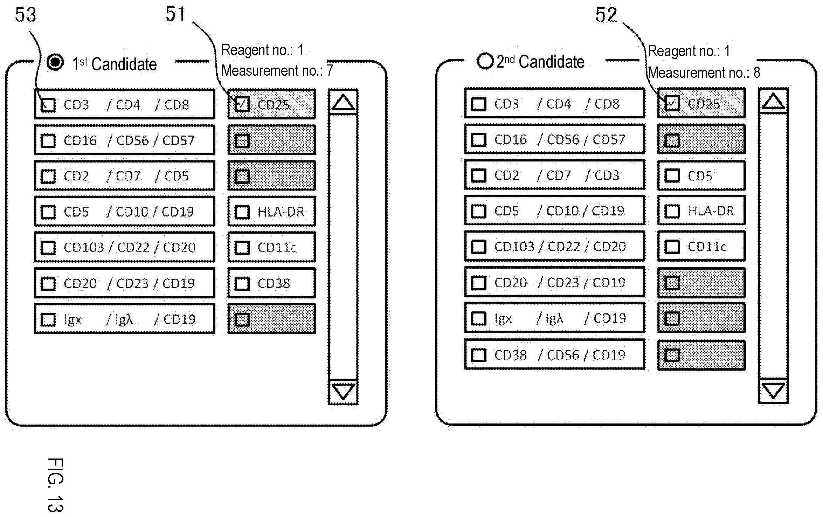

FIG. 13 is an example of screen display of the reagent selection support apparatus;

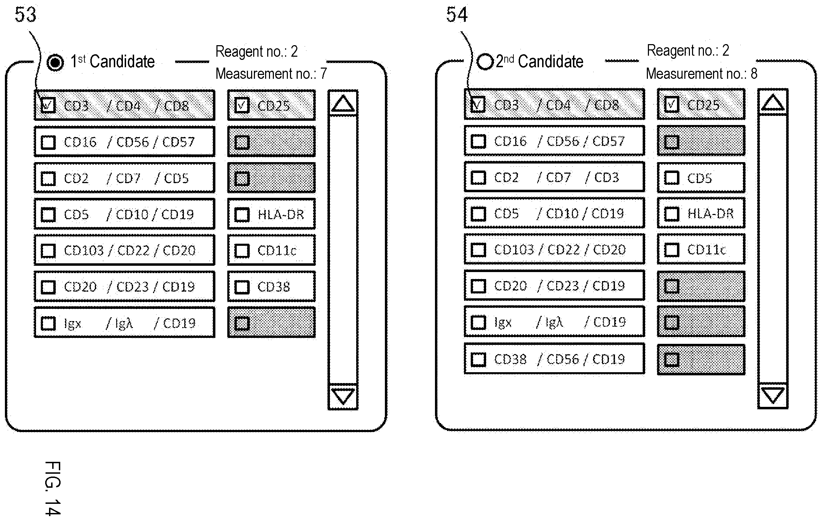

FIG. 14 is an example of screen display of the reagent selection support apparatus;

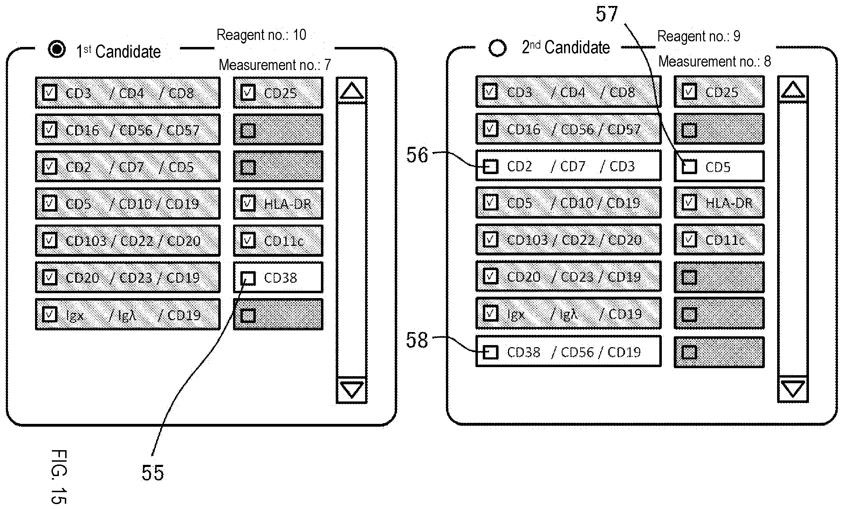

FIG. 15 is an example of screen display of the reagent selection support apparatus;

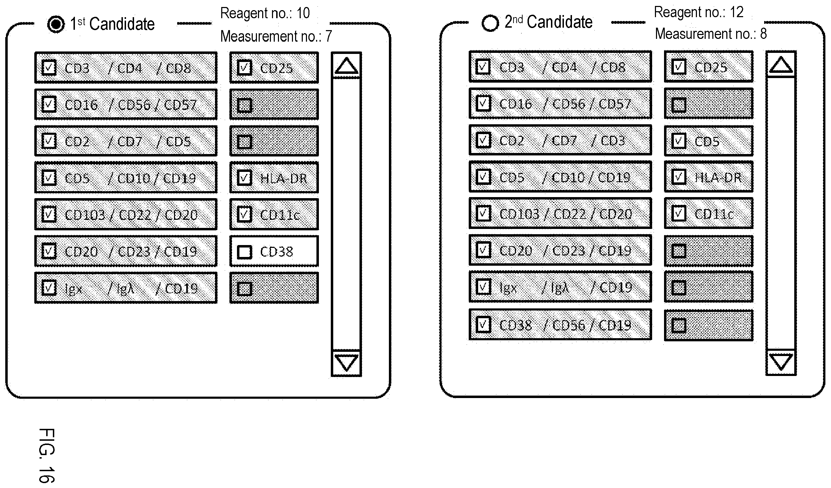

FIG. 16 is an example of screen display of the reagent selection support apparatus;

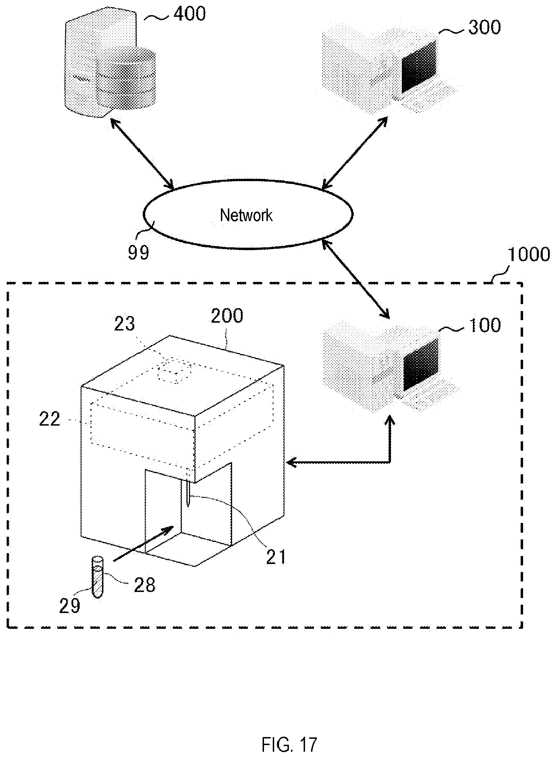

FIG. 17 is a schematic diagram describing an inspection system according to a second embodiment; and

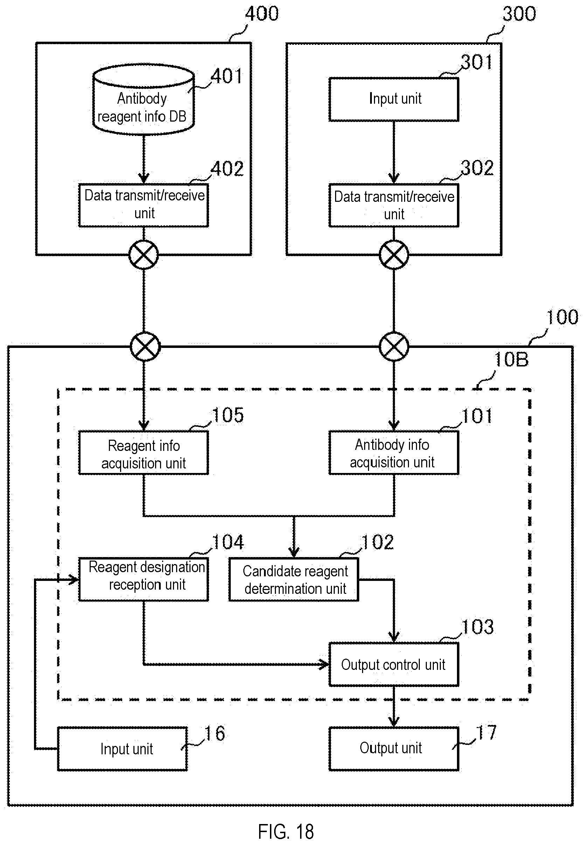

FIG. 18 is a block diagram explaining the functions of the examination system according to the second embodiment.

### DESCRIPTION OF THE EMBODIMENTS OF THE INVENTION

Hereinafter, embodiments of the invention will be described in detail with reference to the accompanying drawings. In the following description and drawings, the same reference numerals denote the same or similar constituent elements, and therefore, descriptions of the same or similar constituent elements are omitted.

### Definition of Terminology

In the following description, an antigen is a target to be detected. The antigen is present in the cell and may be present on the cell membrane, in the cell membrane, or in the cell. A plurality of antigens means a plurality of kinds of antigens. The plurality of kinds of antigens are the same or different biomolecules (proteins, sugar chains, lipids, glycoproteins, glycolipids, lipoproteins, nucleic acids and the like), and preferably are different biomolecules.

A cocktail antibody reagent is a reagent containing a mixture of a plurality of kinds of antibodies capable of binding to different or the same antigen. For example, one cocktail antibody reagent may contain 2 to 5 antibodies. Here, each antibody contained in one cocktail antibody reagent binds to one antigen with at least one antibody. Each antibody contained in the cocktail antibody reagent also may bind two or more antibodies to one antigen. The number of antibodies contained in one cocktail antibody reagent is called a cocktail number. The single antibody reagent also is a reagent containing one type of antibody capable of binding to an antigen. Each antibody may be labeled with a fluorochrome. It is preferable that each antibody contained in one cocktail antibody reagent is labeled with a different fluorochrome. Different fluorochromes mean that the peak wavelength of the fluorescence spectrum of each fluorochrome is optically distinguishable. It also is preferable that the antibody contained in the single antibody reagent is labeled with a fluorochrome which is not included in the cocktail antibody reagent used in combination.

Cocktail antibody reagents and single antibody reagents are collectively referred to as antibody reagents. The information of the antibody reagent includes the type of antibody contained in the antibody reagent, the type of the antigen recognized by the antibody, the type of the labeled fluorochrome and the like. Preferably, the information of the antibody reagent includes information of the cocktail antibody reagent and information of a single antibody reagent that can be used in combination with the cocktail antibody reagent.

### SUMMARY OF THE INVENTION

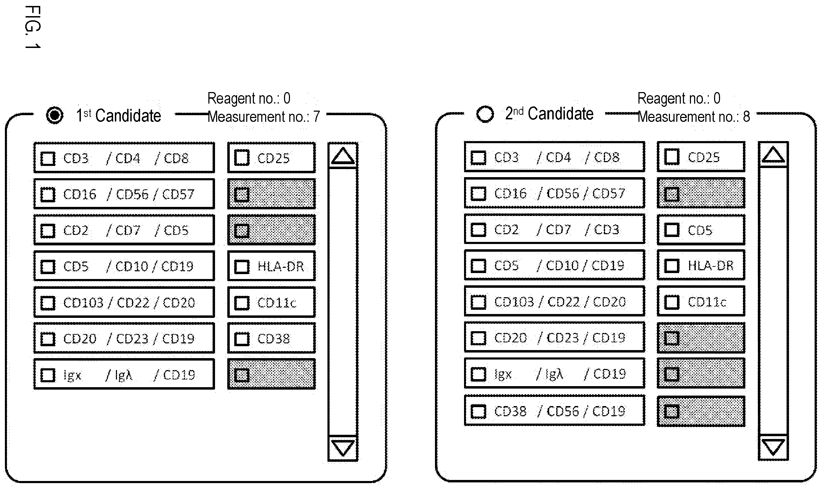

FIG. 1 shows an example of a screen display of the reagent selection support apparatus 100 according to the invention. The reagent selection support apparatus 100 according to the invention automatically determines the candidate combinations of antibody reagents to be used for detecting the antigen designated in the measurement order based on the measurement order and the information of the antibody reagent, and outputs the result in the form exemplified in FIG. 1, for example. The reagent selection support apparatus 100 can determine and present a plurality of combinations of antibody reagent combinations as exemplified as "first candidate" and "second candidate" in FIG. 1. A method for automatically determining candidates for a combination of antibody reagents will be described with reference to the flowcharts of FIGS. 6 to 10.

More specifically regarding the example shown in "first candidate" in FIG. 1, for example, the combination of CD3/CD4/CD8 cocktail antibody reagent and CD25 single antibody reagent shown in the first line is a combination of antibody reagents to be used in a single assay. Also, in the assay shown in the second line, for example, the cocktail antibody reagent of CD16/CD56/CD57 is an antibody reagent to be used in a single assay. A single antibody reagent is not used in the assay shown in the second line. That is, in the example shown in the "first candidate", it is possible to detect all the antigens designated in the measurement order by a total of seven measurements using seven kinds of assays. On the other hand, in the example shown in the "second candidate", it is possible to detect all the antigens designated in the measurement order by a total of eight measurements using eight kinds of assays.

The determination as to which of the combination candidates to actually use among the combinations of the antibody reagent combinations shown in the "first candidate" and "second candidate" can be made interactively, in contrast to the antibody reagent actually stocked at hand. A method of comparing candidates of combinations of antibody reagents determined automatically and antibody reagents actually stocked at hand in an interactive manner is described with reference to the flow chart of FIG. 11 and the examples of screen displays in FIGS. 12 to 16.

The measurement order is, for example, an examination item indicated by a physician or a technician. Preferably, it is an examination item necessary for a physician to judge whether or not a patient suffers from a certain disease. In other words, the measurement order is the type of one or more antigens to be measured in flow cytometry. The information of the antibody reagent is information of the antibody reagent containing the cocktail antibody reagent that can be used by the measurement apparatus body 200 for measuring the sample. In this way the user of the sample measurement apparatus 1000 can efficiently select a combination of appropriate antibody reagents according to the measurement order, even if the user does not have expert knowledge regarding the measurement order or the antibody reagent.

### First embodiment: Summary of Configuration

The examination system according to the first embodiment shown in FIG. 2 includes a sample measurement apparatus 1000. The sample measurement apparatus 1000 also may be connected to a client terminal 300 via a network 99. A measurement order instruction issued by, for example, a physician via the client terminal 300 is transmitted to the sample measuring apparatus 1000 through the network. The client terminal 300 is configured by a general-purpose computer having, for example, a CPU and a memory. The sample (for example, blood) to be examined is separately delivered to the user of the sample measurement apparatus 1000.

The sample measurement apparatus 1000 includes a reagent selection support apparatus 100 and a measurement apparatus body 200 that measures a sample.

The reagent selection support apparatus 100 is configured by, for example, a general-purpose computer, and determines an appropriate combination of antibody reagents according to a measurement order based on a procedure shown in a flowchart to be described later. Based on the combination of antibody reagents determined by the reagent selection supporting apparatus 100, the user of the sample measurement apparatus 1000 prepares the sample 29 by mixing the antibody reagent with the sample to be measured, and supplies the sample 29 to the measurement apparatus body 200 for measurement.

The measurement apparatus body 200 includes a suction unit 21, a fluid circuit 22, and a detection unit 23. In the measurement apparatus body 200, the sample 29 stored in the sample container 28 is suctioned by the suction unit 21, the suctioned sample 29 is fluid-transported by the fluid circuit 22, and the transported sample 29 is measured by the detection unit 23.

In the embodiment, the reagent selection support apparatus 100 concurrently controls the measurement apparatus body 200. That is, the optical information detected by the detection unit 23 is transmitted to the reagent selection support apparatus 100, and the reagent selection support apparatus 100 performs analyses corresponding to the number of cells and each antigen based on the optical information transmitted from the detection unit 23. The reagent selection support apparatus 100 records in advance a computer program defining a processing procedure for controlling the measurement apparatus body 200, and a processing procedure for measuring the measurement value transmitted from the detection section 23 to a recording unit 13 which is described later. The reagent selection support apparatus 100 controls the measurement apparatus body 200 by executing a computer program by the CPU 11 described later.

The suction unit 21 is, for example, a nozzle that can suction and discharge a sample or the like. The fluid circuit 22 is a fluid flow path, and the fluid is transported by, for example, a syringe pump. The sample measurement apparatus 1000 is, for example, a flow cytometer. The flow cytometer measures the sample optically by a flow cytometric method. The flow cytometer can simultaneously measure a plurality of kinds of fluorescence emitted from a sample, and it is possible to shorten the measurement time, by using a cocktail antibody reagent as an antibody reagent.

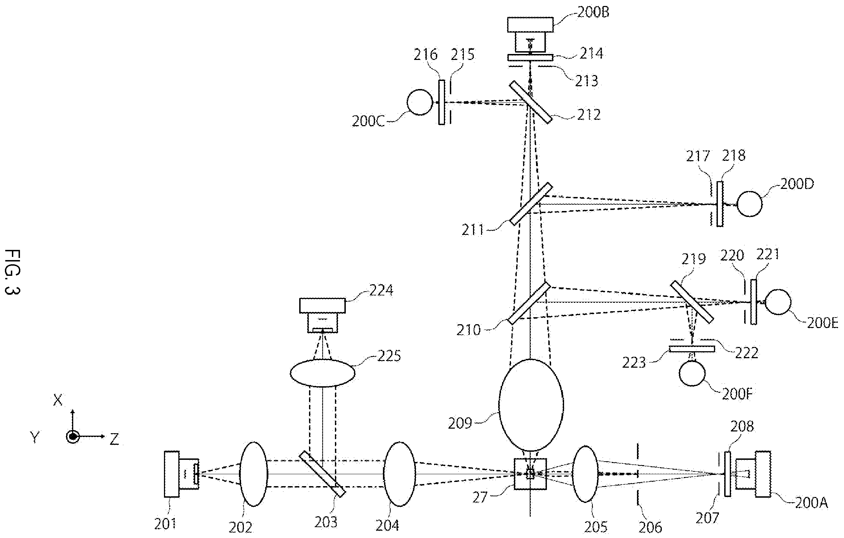

The number of light-receiving elements (for example, photomultiplier tubes) for fluorescence detection provided in the detection unit 23 is determined according to the number of cocktails of the cocktail antibody reagent that the measurement apparatus body 200 can deal with. For example, if the measurement apparatus body 200 is compatible with a cocktail antibody reagent having a cocktail number of "3", the measurement apparatus body 200 includes a total of four light receiving elements for fluorescence detection in the detection unit 23. In this way the detection unit 23 simultaneously measures, for each assay, a total of four color fluorescences composed of the three color fluorescences of the cocktail antibody reagent, and single color fluorescence a single antibody reagent. Hereinafter, the optical system of the flow cytometer will be described referring to FIG. 3, by taking as an example a case where fluorescence of four colors in total are measured at the same time.

FIG. 3 shows an example of the optical system of the flow cytometer as an example of the detection unit 23. The flow cytometer includes a cell 27 for receiving a cell-containing liquid containing cells in the sample, light sources 201 and 224 for irradiating light on particles passing through the cell 27, and light receiving elements 200A to 200F for detecting optical information of light derived from cells and outputting detection signals converted into an electric signals. Here, the sample is a suspension of cells suctioned by a flow cytometer. In the following description of the flow cytometer of FIG. 3, a case where the sample is a mixture of blood and antibody reagent will be described as an example.

It is preferable that the cells emit one or more lights when irradiated with predetermined light. The light given off from cells when the cells are irradiated with predetermined light is collectively referred to as light derived from cells. Light derived from the cells includes scattered light and luminescence. The light derived from the cell may be light of any wavelength, but it is preferably light having a peak wavelength in the range of 400 nm to 850 nm. More specifically, the light derived from the cells is preferably fluorescent light. The light derived from the cells may be light given off by the substance contained in the cells themselves. Alternatively, light derived from the cells may be labeled with a luminescent substance such as a fluorochrome, and the light emitted by the luminescent substance may be detected as light derived from the cells. It also is preferable that the peak wavelength of light derived from cells is different for each antigen. In the first embodiment, the cell-derived fluorescence is derived from a fluorochrome that labels each antibody contained in the detection reagent.

The cell-containing liquid is a liquid containing a cell suspension suctioned from the sample into the flow cytometer, and contains a diluent as necessary. The optical information is information included in one or two or more light wavelength spectra emitted from the cell. The individual light wavelengths and light wavelength regions included in the light wavelength spectrum, and the intensities of the respective light wavelengths or the intensities of the light wavelength regions are included in the light wavelength spectrum. Individual light wavelengths and wavelength regions can be specified by which one of the one or more light receiving elements (described later) receives the light. The intensity of each light wavelength or light wavelength region also can be specified by an electric signal output from the light receiving element that received light.

Hereinafter, the case where the light derived from the cell is scattered light and fluorescence will be specifically described as an example. The light emitted from the light source 201 irradiates the cell 27 through the collimator lens 202, the dichroic mirror 203, and the condenser lens 204. The forward scattered light of the light derived from the cell passing through the cell 27 is collected by the condenser lens 205, and enters the light receiving element 200A through the beam stopper 206, the pinhole plate 207, and the band pass filter 208.

On the other hand, side scattered light and lateral fluorescence of light derived from cells passing through the cell 27 are collected by the condenser lens 209. The side scattered light passes through the dichroic mirrors 210, 211, 212, the pinhole plate 203, and the bandpass filter 214, and enters the light receiving element 200B. The side fluorescence having a wavelength of 520 nm or more and 542 nm or less passes through the dichroic mirrors 210 and 211, reflected by the dichroic mirror 212, and enters the light receiving element 200C through the pinhole plate 215 and the bandpass filter 216. The side fluorescence having a wavelength of 570 nm or more and 620 nm or less passes through the dichroic mirror 210, reflected by the dichroic mirror 211, and enters the light receiving element 200D through the pinhole plate 217 and the bandpass filter 218. Side fluorescence having a wavelength of 670 nm or more and 800 nm or less also is reflected by the dichroic mirror 210, passes through the dichroic mirror 219, and enters the light receiving element 200E through the pinhole plate 220 and the bandpass filter 221.

The light emitted from the light source 224 irradiates the cell 27 through the collimator lens 225, the dichroic mirror 203, and the condenser lens 204. The side fluorescence of the light derived from the cells passing through the cell 27 is collected by the condenser lens 209. Lateral fluorescent light of 662.5 nm or more and 687.5 nm or less is reflected by the dichroic mirror 210, reflected by the dichroic mirror 219, and thereafter enters the light receiving element 200F through the pinhole plate 222 and the bandpass filter 223.

In the example shown in FIG. 3, a laser diode with a wavelength of 488 nm is used for the light source 201 and a laser diode with a wavelength of 642 nm is used for the light source 224. A sheath flow cell is used for the cell 27. A photodiode is used for the light receiving element 200A that receives the forward scattered light, and an avalanche photodiode (APD) is used for the light receiving element 200B that receives the side scattered light. A photomultiplier tube (Photo Multiplier Tube (PMT) is used for the light receiving elements 200C to 200F which receive side fluorescence.

As described above, there are four light receiving elements 200C to 200F for receiving side fluorescence in the flow cytometer shown in FIG. 3. Therefore, the flow cytometer shown in this example has four light receiving elements for detecting fluorescence, and the flow cytometer can simultaneously measure a total four colors of fluorescence including the fluorescence of three colors by the cocktail antibody reagent and the fluorescence of one color by the single antibody reagent per each assay.

The respective detection signals output from the respective light receiving elements 200A to 200F are amplified by an amplification circuit (not shown), converted into digital data by A/D conversion in an A/D converter (not shown). In the present embodiment, the detection signal converted to digital data is transmitted to the reagent selection support apparatus 100, and cell analysis is performed. The amplification circuit is a known amplification circuit configured by, for example, an operational amplifier or the like.

The number of light sources may be one, or two or more. The light source is selected according to the wavelength region of light derived from the cell. When there are two or more light sources, it is preferable that these light sources emit light having different peak wavelengths.

The number of photodiodes, dichroic mirrors, and bandpass filters can be varied according to the number of peak wavelengths of light originating from the cells. The types of the photodiode, the dichroic mirror, and the bandpass filter can also be selected according to the peak wavelength of light derived from the cell, or the wavelength region, and its intensity.

The reagent selection support apparatus 100 transmits information related to detection sensitivity when the detection unit 23 detects scattered light and fluorescence, information related to fluorescence correction according to the combination of detected fluorescence, and information related to gating for selecting a distribution region of cells to be detected to the detection unit 23, and the detection unit 23 performs controls so that appropriate optical information can be acquired according to the antigen based on these pieces of information.

### Operation Summary

In the first embodiment, the information of the antibody reagent that can be used by the measurement apparatus body 200 is recorded in advance in the reagent selection support apparatus 100. First, for example, a physician who examines a patient inputs to the client terminal 300 the measurement order necessary for determining whether the patient is suffering from a certain disease (for example, leukemia). The client terminal 300 transmits the input measurement order to the reagent selection support apparatus 100. The reagent selection support apparatus 100 determines the candidate for the combination of the antibody reagents to be used to detect the antigen designated in the measurement order and outputs the result based on the obtained measurement order and information of antibody reagents including cocktail antibody reagents that can be used by the measurement apparatus body 200. In this way the user of the sample measurement apparatus 1000 can select an appropriate combination of antibody reagents according to the measurement order.

The user also inputs the information of the cocktail antibody reagent stocked at hand and the information of the single antibody reagent to the reagent selection support apparatus 100. As a result, the user can reliably grasp interactively whether a measurement satisfies the measurement order using the antibody reagent stocked at hand while referring to the results of the candidate combination of the antibody reagents determined by the reagent selection support apparatus 100.

Thereafter, having ascertained that measurement that satisfies the measurement order is possible, the user mixes the antibody reagent with the sample 29 for each combination, that is, for each assay on the basis of the combination of the antibody reagents stocked at hand, and sets the sample in the measurement apparatus body 200 for measurement. The measurement apparatus body 200 detects the antigen designated by the measurement order, and the examination result is transmitted to the client terminal 300.

### Hardware Configuration

The reagent selection support apparatus 100 shown in FIG. 4 includes processing unit 10 (10A, 10B), an input unit 16, and an output unit 17.

The processing unit 10 includes a CPU (Central Processing Unit) 11 that performs data processing to be described later, a memory 12 used as a work area for data processing, a recording unit 13 that records programs and processing data described later, a bus 14 for transmitting data, and an interface unit 15 (hereinafter referred to as "I/F unit") for inputting/outputting data to/from an external device. The input unit 16 and the output unit 17 are connected to the processing unit 10. Illustratively, the input unit 16 and the output unit 17 are integrated, and can be configured as a touch panel type input display device.

In order to perform the processing of each step described below with reference to FIGS. 6 to 11, the processing section 10 stores a program according to the invention in an executable form (for example, generated by conversion from a programming language by a compiler) in advance in the recording unit 13, and the processing unit 10 performs processing using the program recorded in the recording unit 13.

In the following description, unless otherwise specified, the processing performed by the processing unit 10 refers to processing that is actually performed by the CPU 11 of the processing unit 10 based on the program stored in the recording unit 13 or the memory 12. The CPU 11 temporarily stores necessary data (such as intermediate data being processed) in the memory 12 as a work area, and appropriately records data to be stored for a long term, such as calculation results, in the recording unit 13.

### Functional Blocks

The processing unit 10A of the reagent selection support apparatus 100 according to the first embodiment shown in FIG. 5 includes an antigen information acquisition unit 101, a candidate reagent determination unit 102, an output control unit 103, and a reagent designation reception unit 104. These functional blocks are realized by installing the program according to the invention in the recording unit 13 or the memory 12 of the processing unit 10A and executing the program by the CPU 11.

In the first embodiment, an antibody reagent information database 401 (hereinafter referred to as "antibody reagent information DB 401 ") is prerecorded in the recording unit 13 or the memory 12 of the processing unit 10A. The antibody reagent information DB 401 is information of an antibody reagent containing a cocktail antibody reagent that can be used by the measurement apparatus body 200.

The client terminal 300 includes an input unit 301 that accepts input of a measurement order, and a data transmission and reception unit 302 that transmits the measurement order to the reagent selection support apparatus 100.

### Method for Determining Candidate Combinations of Antibody Reagents

A method for determining a combination of antibody reagents performed by the reagent selection support apparatus 100 will be described by way of example as a case in which the patient is suspected of suffering from leukemia.

Table 1-1 to Table 1-3 (collectively referred to as "Table 1" below) show examples of information of antibody reagents recorded in the antibody reagent information DB 401 used in the first embodiment.

**Table 1-1**

| No. Cell line | | Cocktail antibody | | | Single antibody |
|---|---|---|---|---|---|
| 1 | T/B/Myeloid | cyCD3 | cyCD79a | cyMPO | CD45 |
| 2 | Stern/Progenitor | CD 117 | CD34 | CD45 | CD56 |
| | | | | | HLA-DR |
| | | | | | CD38 |
| | | | | | CD11b |
| | | | | | CD33 |
| | | | | | CD7 |
| 3 | | CD38 | CD17 | CD45 | CD34 |
| 4 | Myeloid | CD13 | CD33 | CD45 | CD16 |
| | | | | | CD11b |
| | | | | | CD64 |
| | | | | | CD36 |
| | | | | | CD14 |
| | | | | | CD34 |
| | | | | | CD15 |
| | | | | | CD7 |
| | | | | | CD2 |
| 5 | | CD15 | HLA-DR | CD45 | CD13 |
| 6 | Monocytic | CD14 | CD64 | CD45 | HLA-DR |
| | | | | | CD36 |
| | | | | | CD16 |
| | | | | | CD13 |
| | | | | | CD15 |
| | | | | | CD2 |
| | | | | | CD4 |
| 7 | | CD11c | CD11b | CD45 | CD14 |
| | | | | | CD16 |
| | | | | | CD13 |
| | | | | | CD15 |

**Table 1-2**

| No. | Cell line | Cocktail antibody | | | Single antibody |
|---|---|---|---|---|---|
| 8 | Megakaryocytic | CD41 | CD61 | CD45 | CD42b |
| | | | | | CD13 |
| | | | | | CD33 |
| | | | | | HLA-DR |
| | | | | | CD34 |
| 9 | Erythrocytic | CD235a | CD36 | CD45 | CD71 |
| | | | | | CD33 |
| | | | | | HLA-DR |
| 10 | | CD5 | CD10 | CD19 | CD45 |
| | | | | | CD23 |
| | | | | | CD20 |
| | | | | | CD34 |
| | | | | | CD79a |
| | | | | | HLA-DR |
| 11 | | CD103 | CD22 | CD20 | CD45 |
| | B cell | | | | CD24 |
| | | | | | CD19 |
| | | | | | CD10 |
| | | | | | CD 23 |
| | | | | | CD11c |
| 12 | | CD20 | CD23 | CD19 | CD45 |
| | | | | | CD22 |
| | | | | | CD238 |
| 13 | | Ig *κ* | Ig *λ* | CD19 | CD45 |
| | | | | | IgM |
| | | | | | CD5 |
| 14 | Plasma | CD38 | CD56 | CD19 | CD45 |
| | | | | | Ig *κ* |
| | | | | | Ig *λ* |
| | | | | | CD138 |
| | | | | | CD20 |
| | | | | | CD117 |
| | | | | | CD20 |

**Table 1-3**

| No. | Cell line | Cocktail antibody | | | Single Antibody |
|---|---|---|---|---|---|
| 15 | T/NK cell | CD2 | CD7 | CD5 | CD45 |
| | | | | | CD3 |
| | | | | | CD1a |
| 16 | | CD2 | CD7 | CD3 | CD45 |
| | | | | | CD5 |
| | | | | | CD25 |
| | | | | | CD19 |
| | | | | | CD1a |
| | | | | | CD56 |
| | | | | | CD10 |
| 17 | | CD3 | CD4 | CD8 | CD45 |
| | | | | | CD19 |
| | | | | | CD56 |
| 18 | | CD16 | CD56 | CD57 | CD45 |
| | | | | | CD3 |

The information of the antibody reagents shown in Table 1 includes information of cocktail antibody reagents and information of single antibody reagents that can be used in combination with the cocktail antibody reagents. In the following description, the variable N is used to specify the number of cocktails of cocktail antibody reagents. The variable N is a natural number (positive non-zero integer). In the example shown in Table 1, the cocktail number of the cocktail antibody reagent is "3", and the value of the variable N is "3". In the following description, candidates for combinations of antibody reagents are determined based on information of antibody reagents containing a cocktail antibody reagent having a cocktail number of "3", but the number of cocktails of cocktail antibody reagents is not limited to this number. The cocktail number of the cocktail antibody reagent to be used can be appropriately changed according to the cocktail number of the cocktail antibody reagent that the measuring apparatus main body 200 can support in one assay. Please refer to the "Additional Information" section described in detail below.

In the following description, the variables x and y are used to specify the cocktail antibody reagent and the single antibody reagent in the information of the antibody reagent shown in Table 1 or within the candidates of the antibody reagent. The variables x and y are natural numbers (positive non-zero integers).

In the following description, the cocktail antibody reagent (x) means the x^{th} cocktail antibody reagent. For example, in the information of the antibody reagent shown in Table 1, the cocktail antibody reagent (2) is a cocktail antibody reagent shown in "No. 2" in Table 1 in which three antibodies CD 117, CD 34 and CD 45 are collected. The single antibody reagent (y) means the y^{th} single antibody reagent. For example, in the information of the antibody reagent shown in Table 1, the single antibody reagent (3) which can be used in combination with the cocktail antibody reagent (2) is the third "CD38" antibody among the seven single antibody reagents "CD56, HLA-DR, CD38, CD11b, CD33, CD7, CD34" that can be used in combination with the cocktail antibody reagent indicated by "No. 2" in Table 1.

The measurement order used for illustrative purposes in the first embodiment is as follows. Measurement order: "HLA-DR, CD38, CD10, CD11c, CD19, CD20, CD22, CD23, CD103, IgK, Igλ, CD2, CD3, CD4, CD8, CD5, CD7, CD25, CD16, CD56"

The processing unit 10A of the reagent selection support apparatus 100 performs the operations shown in the flowcharts of FIGS. 6 to 10. When explaining the operations using each function block shown in FIG. 5, the process of step S1 is performed by the antigen information acquisition unit 101. The candidate reagent determination unit 102 performs the processes from steps S2 to S5, steps S11 to S20, steps S151 to S158, and steps S161 to S170, excluding steps S1 and S6. The process of step S6 is performed by the output control unit 103.

Refer to the flowchart of FIG. 6. In step S1, the processing unit 10A acquires a measurement order from the client terminal 300. The measurement order is transmitted from the data transmission/reception unit 302 in the form of text information, for example.

In step S2, the processing unit 10A sets an initial value "1" to the variable x, and first selects the first cocktail antibody reagent (1).

In step S3, the processing unit 10A determines whether there are one or more antibodies contained in the x^{th} cocktail antibody reagent (x) in the measurement order. If the antibody exists, the processing unit 10A performs the processing of step S11 described later, and if not, the determination of step S4 is performed.

That is, in order to present a plurality of candidate combinations of antibody reagents through the repetitive processes of steps S3 to S5, the processing unit 10A first selects the first cocktail antibody reagent (1), and a determination is made as to whether this first cocktail antibody reagent (1) is a reagent candidate. In the case of being a reagent candidate, the processing unit 10A carries out the processing of steps S11 to S20 described with reference to FIG. 7, and performs a process to determine the degree of matching with the antigen of the measurement order, and a process of determining whether to use the candidate in combination with a single antibody reagent. In the case of not being a reagent candidate, the processing unit 10A performs the processing of steps S4 to S5, and similarly determines whether the second cocktail antibody reagent (2) is a reagent candidate in step S3.

Specifically, through the repetition of steps S3 to S5, eleven cocktail antibody reagents indicated by variables x = 3, 5, and 10 to 18 among the 18 cocktail antibody reagents shown in Table 1, becomes a cocktail antibody reagent initially extracted from the measurement order, as shown in step S11. That is, it can be said that the process in step S3 is the process of determining the cocktail antibody reagent (x) to be extracted initially from the measurement order in step S11. When the cocktail antibody reagent to be initially extracted from the measurement order changes, the candidates for the combination of the antibody reagent also changes because the candidates for the cocktail antibody reagent to be extracted from the measurement order next are limited. This ultimately presents multiple candidates for antibody reagents.

In step S4, the processing unit 10A determines whether the x+1^{th} cocktail antibody reagent (x+1) exists in the antibody reagent information DB 401. If the reagent exists, the processing unit 10A increases the variable x by "1" in step S5, and then makes the determination in step S3. If there is no such information, the processing unit 10A performs the process of step S6 since the determination process has been completed for all the cocktail antibody reagents in the antibody reagent information DB 401.

In step S6, the processing unit 10A displays candidates for a combination of antibody reagents determined as reagent candidates. The display order of the combination of antibody reagents displayed by the processing unit 10A as the determined result is arbitrary. For example, the processing unit 10A can display a plurality of candidates of the determined combination of antibody reagents in descending order of the number of reagents to be used or the number of measurements. Alternatively, the processing unit 10A can display a plurality of candidates of the determined combination of antibody reagents in descending order of evaluation points to be described later.

Refer to the flowchart of FIG. 7. The processing in steps S11 to S20 shown in FIG. 7 is processing performed in step S3 when there is at least one antibody contained in the x^{th} cocktail antibody reagent (x) in the measurement order.

In step S11, the processing unit 10A updates the measurement order by removing the antibody contained in the cocktail antibody reagent (x) from the measurement order. The antibody removed from the measurement order is the antibody contained in the cocktail antibody reagent (x), which was determined to be present in the measurement order in step S3.

In step S12, the processing unit 10A adds the cocktail antibody reagent (x) to a new reagent candidate.

In step S13, the processing unit 10A adds the number of antibodies matching the measurement order to the new evaluation point. The number of antibodies matching the measurement order is the number of antibodies contained in the cocktail antibody reagent (x), which was determined to be present in the measurement order in step S3.

In step S14, the processing unit 10A sets an initial value "3" to the variable N representing the cocktail number of the cocktail antibody reagent.

In step S15, the processing unit 10A performs a cocktail antibody reagent search process. The cocktail antibody reagent search process will be described later with reference to FIG. 8. If the value of the variable N is "3 ", the cocktail antibody reagent search process is a process of first determining whether all three antibodies among the three antibodies of the cocktail antibody reagent (x) are included in the measurement order.

In step S16, the processing unit 10A performs a single antibody reagent search process. The single antibody reagent search process is a process of determining a single antibody reagent that can be used in combination with the cocktail antibody reagent (x). The single antibody reagent search process will be described later with reference to FIGS. 9 and 10.

In step S17, the processing unit 10A determines whether an antibody still exists in the measurement order.

If an antibody exists, in step S18, the processing unit 10A records the new reagent candidate added in step S12 and the new evaluation point added in step S13, and performs the determination in step S4.

If the antibody does not exist, the processing unit 10A determines whether the variable N can be subtracted in step S19, and then subtracts "1" from the variable N in step S20. Thereafter, the processing unit 10A performs the cocktail antibody reagent search process in step S15 with the value of the variable N after the subtraction. If the value of the variable N is "2", the cocktail antibody reagent search process determines whether two antibodies among the three antibodies of the cocktail antibody reagent (x) are included in the measurement order.

Refer to the flowchart of FIG. 8. The processing of steps S151 to S158 shown in FIG. 8 is a cocktail antibody reagent search processing.

In step S151, the processing unit 10A sets an initial value "1" to the variable x, and first selects the first cocktail antibody reagent (1).

In step S152, the processing unit 10A searches the measuring order for the x^{th} cocktail antibody reagent (x), and in step S153 the processing unit 10A determines whether there are N or more antibodies contained in the cocktail antibody reagent (x) in the measurement order. The processing unit 10 A performs the processing of step S154 if N or more antibodies are present, and the determination in step S157 is performed if there are not N antibodies.

In step S154, the processing unit 10A removes the antibody contained in the cocktail antibody reagent (x) from the measurement order, and updates the measurement order. The antibody removed from the measurement order is N antibodies contained in the cocktail antibody reagent (x), which was determined to be present in the measurement order in step S153.

In step S155, the processing unit 10A adds the cocktail antibody reagent (x) to the reagent candidate.

In step S156, the processing unit 10A adds the value N to the evaluation point. After the end of step S156, the processing of step S15 is called recursively, and the processing section 10A continues the processing of step S15.

In step S157, the processing unit 10A determines whether the x+1^{th} cocktail antibody reagent (x+1) exists in the antibody reagent information DB 401. If the antibody exists, the processing unit 10A increases the variable x by "1" in step S158, and then makes the determination in step S153. If the antibody does not exist, the processing unit 10A terminates the cocktail antibody reagent search process of step S15. At the end of step S15, the cocktail antibody reagent of the reagent candidate, and the remainder of the measurement order at that time are listed. After finishing the process of step S15, the processing unit 10A performs the process of step S16 shown in FIG. 7.

Refer to the flowcharts of FIGS. 9 and 10. The processing of steps S161 to S170 shown in FIGS. 9 and 10 is a single antibody reagent search process.

In step S161, the processing unit 10A sets the initial values "1" to the variables x and y, respectively. The variable x indicates the first cocktail antibody reagent of the reagent candidate, and the variable y indicates the first single antibody reagent that can be used in combination with the cocktail antibody reagent (x).

In step S162, the processing unit 10A searches the measurement order for the y^{th} single antibody reagent (y), and in step S163, the processing unit 10A determines whether the antibody indicated in the single antibody reagent (y) is present in the measurement order, as shown in FIG. If the antibody is in the measurement order, the processing unit 10A performs the process of step S164, and if not, the determination of step S167 is performed.

In step S164, the processing unit 10A removes the antibody indicated by the single antibody reagent (y) from the measurement order and updates the measurement order.

In step S165, the processing unit 10A adds the single antibody reagent (y) to the reagent candidate.

In step S166, the processing unit 10A adds the value "1" to the evaluation point. After the end of step S166, the process of step S16 is called recursively, and the processing unit 10A continues the process of step S16.

In step S167, the processing unit 10A determines whether the y+1^{th} single antibody reagent (y + 1) exists in the antibody reagent information DB 401 for the x^{th} cocktail antibody reagent (x). If antibody reagent exists, the processing unit 10A increases the variable y by "1" in step S168, and then makes the determination in step S163. If the antibody reagent does not exist, the processing unit 10A performs the determination of step S169.

In step S169, the processing unit 10A determines whether the x+1^{th} cocktail antibody reagent exists in the reagent candidate. If the cocktail antibody reagent exists, the processing unit 10A increases the variable x by "1" in step S170, and then performs the determination in step S163. If the cocktail antibody reagent does not exist, the processing unit 10A terminates the single antibody reagent search processing of step S16. At the end of step S16, the cocktail antibody reagent of the reagent candidate and the single antibody reagent of the reagent candidate and the remaining measurement orders at the present time are listed. After finishing the processing of step S16, the processing unit 10A performs the determination of step S17 shown in FIG. 7.

After the process of step S17, the reagent candidates and the evaluation points recorded in step S18 are output to the output unit 17 of the reagent selection support apparatus in step S6, for example, as shown in FIG. 12. In the example shown in FIG. 12, the processing unit 10A displays a combination of reagent candidates with the highest evaluation point on the left side of the screen as a first candidate, and a combination of reagent candidates with the second highest evaluation point of 2 on the right side of the screen.

As described above, the user of the reagent selection support apparatus 100 can mix the antibody reagent to the sample 29 based on the combination of the antibody reagents determined by the reagent selection support apparatus 100, and set the antibody reagent in the measurement apparatus main body 200 for measurement. In this way, the user can select an appropriate combination of antibody reagents according to the measurement order.

### Interactive Method for Supporting Selection of Antibody Reagent

The user can also input the information of the cocktail antibody reagent stocked at hand and the information of the single antibody reagent to the reagent selection support apparatus 100. In this way the user can interactively grasp whether the measurement satisfying the measurement order can be performed using the antibody reagent stocked at hand while referring to the candidate of the determined combination of antibody reagents. Hereinafter, the description will be made with reference to the flowchart of FIG. 11 and screen display examples of FIGS. 12 to 16.

The processing unit 10A of the reagent selection support apparatus 100 includes an output control unit 103 and a reagent designation reception unit 104. The reagent designation reception unit 104 acquires the designation of the reagent candidate input from the user via the input unit 16, and transmits the candidate to the output control unit 103. The output control unit 103 controls the display mode of the designated reagent candidate at the output unit 17 based on the specification of the reagent candidate received from the reagent designation reception unit 104. The reagent designation reception unit 104 also counts the number of input reagent candidate designations as the number of reagents to be used, and displays the information on the screen. Specifically, the number of designations of reagent candidates corresponds to the number of check boxes checked.

The processing unit 10A of the reagent selection support apparatus 100 detects the input to the check box and performs the operation shown in the flowchart of FIG. 11 each time the input is detected. Explanation will be given using each functional block shown in FIG. 5, the processes in steps S31 and S32 are performed by the reagent designation reception unit 104, and the processes in steps S33 and S34 are performed by the output control unit 103.

Combinations of antibody reagents determined by the reagent selection support apparatus 100 are displayed in FIG. 12. The user confirms the information of the cocktail antibody reagent stocked at hand and the information of the single antibody reagent, and checks the check box of the corresponding column when the antibody reagent stocked at hand is displayed among the combination of antibody reagent displayed in FIG. 12.

For example, if the user has a single antibody reagent of CD25, the user checks the check box 51 corresponding to the single antibody reagent of CD25 in the combination of the first candidate antibody reagent. In step S31, when the processing unit 10A acquires a designation of the reagent candidate for the combination of the first candidate antibody reagents, in step S32 the processing unit 10A determines whether the same designated reagent candidate is included in the combination of the second candidate antibody reagent.

If the second candidate also includes the same designated reagent candidate as the first candidate, the processing unit 10A checks the check box 52 for the second candidate antibody reagent candidate in conjunction with the check of the first candidate check box, as shown in FIG. 13, in step S33. The check box 52 is a check box corresponding to the single antibody reagent of CD25 within the combination of the second candidate antibody reagent. When the second candidate does not include the same reagent candidate specified as the first candidate, the processing unit 10A terminates the process. Upon detecting the input to the check box, the processing unit 10A again performs the processing from step S31.

In step S34, the processing unit 10A changes the display manner of the check boxes 51 and 52 that are checked. The changed display mode is, for example, a hatched display.

Hereinafter, the user confirms the information of the cocktail antibody reagent and the information of the single antibody reagent which are stocked at hand, and checks the check box in the corresponding column when the antibody reagent stocked at hand is displayed in the combination of the antibody reagents displayed in FIG. 13.

For example, when one has a cocktail antibody reagent of CD3/CD4/CD8, the user checks the check box 53 corresponding to the cocktail antibody reagent of CD3/CD4/CD8 within the combination of the first candidate antibody reagent. This cocktail antibody reagent of CD3/CD4/CD8 is also included in the second candidate. Therefore, as shown in FIG. 14, the processing unit 10A also checks the check box 54 in conjunction with the second candidate antibody reagent candidate. The check box 54 is a check box corresponding to the cocktail antibody reagent of CD3/CD4/CD8 within the second candidate antibody reagent combination. The check boxes 53 and 54 are hatched.

Similarly, for the combination of the first candidate antibody reagent, the user confirms the information on the cocktail antibody reagent stocked at hand and the information of the single antibody reagent, and inputs the information to the reagent selection support apparatus 100.

In the state shown in FIG. 15, the measurement order to be detected is a state in which one "CD38" remains.

For example, if the user has CD38 single antibody reagent, the user can check the check box 55. In this way, all the combinations of antibody reagents indicated in the first candidate are checked and hatched. That all antibody reagents are displayed in hatching in the first candidate means that measurements satisfying the measurement order are possible using the cocktail antibody reagent and the single antibody reagent which the user stocks at hand. Thereafter, the user can detect the antibody designated in the measurement order using the measurement apparatus body 200 based on the combination of the antibody reagents indicated in the first candidate.

On the other hand, if the user does not have the CD38 single antibody reagent, the user cannot check the check box 55. In such a case, the user confirms the information on the cocktail antibody reagent stocked on hand and the information on the single antibody reagent for the combination of antibody reagents indicated in the second candidate. For example, if the user has a cocktail antibody reagent of CD2/CD7/CD3, the user can check the check box 56 corresponding to the cocktail antibody reagent of CD2/CD7/CD3 in the second candidate antibody reagent combination.

Hereinafter, similarly, regarding the combination of the second candidate antibody reagent of the second candidate, the user confirms the information of the cocktail antibody reagent stocked at hand and the information of the single antibody reagent, and inputs it to the reagent selection support apparatus 100.

In the state shown in FIG. 15, for example, if the user has the CD5 single antibody reagent and the CD38/CD56/CD19 cocktail antibody reagent, the user can check the check boxes 57 and 58. In this way, as shown in FIG. 16, all combinations of antibody reagents shown in the second candidate are checked and hatched. That all the antibody reagents shown in the second candidate are displayed in hatching means that measurements satisfying the measurement order are possible, that is, it is possible to perform the measurement using the cocktail antibody reagent and the single antibody reagent stocked by the user at hand. Thereafter, the user can detect the antibody designated in the measurement order using the measurement apparatus body 200 based on the combination of the antibody reagents indicated in the second candidate.

As described above, the user of the reagent selection support apparatus 100 can also input the information of the cocktail antibody reagent stocked at hand and the information on the single antibody reagent to the reagent selection support apparatus 100. In this way the user can interactively grasp whether the measurement satisfying the measurement order can be performed using the antibody reagent stocked at hand while referring to the candidate of the determined combination of antibody reagents.

### Second Embodiment

In the first embodiment, the information of the antibody reagent that can be used by the measurement apparatus body 200 is recorded in advance in the reagent selection support apparatus 100. In the second embodiment, the information of the antibody reagent that can be used by the measurement apparatus main body 200 is acquired from the external server 400. The external server 400 is configured by a general-purpose computer having, for example, a CPU and a memory.

The examination system according to the second embodiment shown in FIG. 17 further includes a server 400 in addition to the sample measurement apparatus 1000 and the client terminal 300. The server 400, the sample measurement apparatus 1000, and the client terminal 300 are connected to each other through a network 99.

The processing unit 10B of the reagent selection support apparatus 100 according to the second embodiment shown in FIG. 18 further includes a reagent information acquisition unit 105 in addition to the processing unit 10A according to the first embodiment. That is, the processing unit 10B includes an antigen information acquisition unit 101, a candidate reagent determination unit 102, an output control unit 103, a reagent designation reception unit 104, and a reagent information acquisition unit 105. These functional blocks are realized by installing the program according to the invention in the recording unit 13 or the memory 12 of the processing unit 10B, and executing this program by the CPU 11.

The external server 400 includes a data transmission/reception unit 402 that transmits the antibody reagent information DB 401 to the reagent selection support apparatus 100.

In the second embodiment, the reagent information acquisition unit 105 acquires the antibody reagent information DB 401 from the external server 400, and records the database in the recording unit 13 or the memory 12 of the processing unit 10B. By updating the antibody reagent information DB 401 to be recorded in the external server 400 to the latest state, the reagent selection support apparatus 100 can usually determine combinations of antibody reagent candidates based on the latest antibody reagent information DB 401. The second embodiment is the same as the first embodiment with respect to the method of determining the candidate combination of the antibody reagent, and the method of supporting the selection of the antibody reagent interactively. The processing performed by the reagent information acquisition unit 105, which is a functional block, is actually performed by the processing unit 10B shown in FIG. 4.

According to the inspection system of the second embodiment described above, the information of the antibody reagent that can be used by the measurement apparatus body 200 can normally be maintained in an updated state in addition to the effect of the examination system according to the first embodiment.

### Additional Items

Although the invention has been described with reference to specific embodiments, the present invention is not limited to the above-described embodiments.

In the first and second embodiments, the processing units 10A and 10B are realized as an integrated device, but the processing units 10A and 10B do not need to be integrated devices, and may be a CPU 11, a memory 12, a recording unit 13 and the like are arranged in different locations, and they may be connected by a network. The processing units 10A and 10B, the input unit 16, and the output unit 17 are not necessarily arranged in one place, but they may be arranged separately from each other and connected to each other so as to communicate with each other via a network.

Although the function blocks of the antigen information acquisition unit 101, the candidate reagent determination unit 102, the output control unit 103, the reagent designation reception unit 104, and the reagent information acquisition unit 105 are implemented by a single CPU 11 in the first and second embodiments, it is not necessary for each of these functional blocks to be executed by a single CPU 11, and these functional blocks may be distributedly processed by a plurality of CPUs.

Although the program for performing the processing of each step described in FIG. 6 to FIG. 11 is recorded in advance in the recording unit 13 in the first and second embodiments, the program also may be stored in a storage medium such as a CD, or may be installed from the computer readable non-transitory tangible recording medium (not shown) to the processing units 10A and 10B, or the processing units 10A and 10B may be connected to a network 99 and the program may be downloaded from the external server 400 and installed via the network 99.

Although the input unit 16 and the output unit 17 are integrated and realized as a touch panel type display device in the first and second embodiments, the input unit 16 may be configured with a keyboard, a mouse, or the like, and the output unit 17 may be configured by a liquid crystal display or the like. Alternatively, the output unit 17 may be configured by a printer or the like, and the combination of antibody reagents determined by the reagent selection support apparatus 100 may be printed.

Although the measurement order is transmitted from the client terminal 300 to the reagent selection support apparatus 100 via the network 99 in the first and second embodiments, the measurement order also may be input through the input unit 16 of the reagent selection support apparatus 100. In this case, the measurement order is transmitted to the user of the reagent selection support apparatus 100, for example, by means of e-mail, telephone or the like, and the user inputs the transmitted measurement order to the reagent selection support apparatus 100 through the input unit 16.

In the first and second embodiments, when the measurement apparatus body 200 can identify three or more color fluorochromes, one assay can be performed using, for example, a cocktail antibody reagent having a cocktail number of "3", and the reagent selection support apparatus 100 can determine the candidate combination of antibody reagents based on the information of the antibody reagent containing the cocktail antibody reagent having the cocktail number of "3". The cocktail number of the cocktail antibody reagent is not limited insofar as the cocktail number is 2 or more. For example, when the measurement apparatus body 200 can identify four or more colors of fluorochromes, one assay can be performed using a cocktail antibody reagent with a cocktail number of "4", and the reagent selection support apparatus 100 may determine a combination of antibody reagents based on information of the antibody reagent containing a cocktail antibody reagent having a cocktail number of "4". In this case, the measurement apparatus body 200 has a total of five light receiving elements for fluorescence detection in the detection unit 23, and the detection unit 23 simultaneously measures the fluorescence of five colors in total, that is, the fluorescence of four colors by the cocktail antibody reagent and the fluorescence of one color by the single antibody reagent. For example, when the measurement apparatus body 200 can identify two or more fluorochromes, one assay can be performed using a cocktail antibody reagent having a cocktail number of "2", and the reagent selection support apparatus 100 may determine the combination of antibody reagents based on the information of the antibody reagent containing the cocktail antibody reagent having the cocktail number of "2". In this case, the measurement apparatus body 200 has a total of three light receiving elements for fluorescence detection in the detection unit 23, and the detection unit 23 simultaneously detects fluorescence of two colors by the cocktail antibody reagent, and fluorescence of one color by the single antibody reagent.

Although the display mode after input to the check box when displaying the antibody reagent stocked at hand is indicated by hatching in the first and second embodiments, the display mode is not limited to this configuration. For example, instead of a hatching display, black and white may be inverted and displayed, and the frame of the check box may be emphatically displayed by double lines, for example. That is, any mode may be used insofar as the user can visually grasp the change of the display mode.

In addition to the above-described first and second embodiments, the invention has a third embodiment described below. The detailed explanation of the third embodiment other than mentioned below is the same as in the first and second embodiments.

In a third embodiment of the invention, the reagent selection support apparatus 100 is a reagent selection support apparatus for supporting the selection of a cocktail antibody reagent containing a plurality of antibodies, and includes acquisition units 15 and 16 for acquiring measurement orders for measuring a plurality of antigens, a processing unit 10 for determining a plurality of antibody reagents that can be used in combination with an assay for detecting a plurality of antigens based on the measurement order acquired by the acquisition units 15 and 16, and an output unit 17 for outputting information of a plurality of antibody reagents capable of being used in combination with the assay, the information being determined by the processing unit 10.

The information of a plurality of antigens is referred to as antigen information of 2 or more, 3 or more, 4 or more, 5 or more, 6 or more, 10 or more, 15 or more. The antibody reagent may be a cocktail antibody reagent, a combination of a cocktail antibody reagent and a single antibody reagent, or a combination of different cocktail antibody reagents. Determining a plurality of antibody reagents means determining two or more antibody reagents to be used in one assay. The term "capable of being used in combination" means that at least one fluorochrome contained in the first antibody reagent and at least one fluorochrome contained in the second antibody reagent can be distinguished and detected. The first antibody reagent may be a cocktail antibody reagent or a single antibody reagent. When the first antibody reagent is a cocktail antibody reagent, it is preferable that the second antibody reagent is either a single antibody reagent or a cocktail antibody reagent. When the second antibody reagent is a cocktail antibody reagent, it is preferable that the first antibody reagent is either a single antibody reagent or a cocktail antibody reagent. Both the first antibody reagent and the second antibody reagent may be cocktail antibody reagents. For example, in the case of detection using at least four color fluorochromes in one assay, one assay can be performed in which the first antibody reagent is a cocktail antibody reagent having a cocktail number of "2" or more and the second antibody reagent is a cocktail antibody reagent having a cocktail number of "2" or more. In the case where the first antibody reagent is a single antibody reagent, the second antibody reagent may be used as a cocktail reagent having a cocktail number of "3". Even when the first antibody reagent and the second antibody reagent are combined, if the four measurement items can not be assayed at one time, three measurement items are assayed using the first antibody reagent and the second antibody reagent in combination in the first assay, and the third antibody reagent is selected so as to assay the remaining measurement items in the second assay. The third antibody reagent also may be a cocktail antibody reagent or a single antibody reagent.

### EXPLANATION OF THE REFERENCE NUMERALS

10, 10A, 10B processing unit; 12 memory; 13 recording unit; 14 bus; 15 interface unit; 16 input unit; 17 output unit; 21 suction unit; 22 fluid circuit; 23 detection unit; 27 sheath flow cell; 28 sample container; 29 sample; 99 network; 100 reagent selection support apparatus; 101 antigen information acquisition unit; 102 candidate reagent determination unit; 103 output control unit; 104 reagent designation reception unit; 105 reagent information acquisition unit; 200 measurement apparatus body; 300 client terminal; 301 input unit; 302 data transmission and reception unit; 400 server; 401 antibody reagent information database; 402 data transmission and reception unit; 1000 sample measurement apparatus.

## Claims

1. A reagent selection support apparatus for supporting the selection of a cocktail antibody reagent containing a plurality of antibodies, the apparatus comprising:
an acquisition unit that acquires a measurement order having a plurality of antigens as measurement targets;
a processing unit that determines a plurality of the cocktail antibody reagents to be used in a plurality of assays for detecting the plurality of antigens based on the measurement order acquired by the acquisition unit; and
an output unit that outputs information of the plurality of cocktail antibody reagents used in the plurality of assays, the information being determined by the processing unit.

2. The reagent selection support apparatus according to claim 1, further comprising:
a storage unit for storing information of a plurality of cocktail antibody reagents composed of combinations of different antibodies;
wherein the processing unit determines a plurality of cocktail antibody reagents to be used in the plurality of assays based on the information of the plurality of cocktail antibody reagents read from the storage unit and the measurement order.

3. The reagent selection support apparatus according to claim 2, wherein
each of the plurality of cocktail antibody reagents is configured by a combination of different antibodies.

4. The reagent selection support apparatus of any one of claims 1 to 3, wherein
the processing unit determines an antibody reagent that can be used in combination with the cocktail antibody reagent in at least one of the plurality of assays; and
the output unit outputs information of the antibody reagent that can be used in combination with the cocktail antibody reagent.

5. The reagent selection support apparatus of any one of claims 1 to 4, wherein
the processing unit determines, based on the measurement order, a first candidate to be a combination of cocktail antibody reagents used in the plurality of assays, and a second candidate to be a combination of cocktail antibody reagents different from the first candidate; and
the output unit outputs the first candidate and the second candidate determined by the processing unit.

6. The reagent selection support apparatus according to claim 5, wherein
the output unit outputs the first candidate and the second candidate so as to be mutually distinguishable from each other.

7. The reagent selection support apparatus according to claim 5, wherein
the processing unit accepts the input of a designated cocktail antibody reagent to be used from any one of the first candidate and the second candidate; and
the processing unit changes the display mode of the cocktail antibody reagent corresponding to the designation of each of the first candidate and the second candidate.

8. The reagent selection support apparatus according to any one of claims 5 to 7, wherein
the first candidate and the second candidate are presented according to the number of times of using the cocktail antibody reagent and/or the magnitude relationship of the number of measurements.

9. The reagent selection support apparatus according to claim 8, wherein
the first candidate and the second candidate are presented according to the magnitude relationship of the number of measurements from among the first candidate and the second candidate having the same number of uses of the cocktail antibody reagent, when there are a plurality of results in which the number of uses of the cocktail antibody reagent is the same.

10. The reagent selection support apparatus according to any one of claim 5 to 7, wherein
the processing unit presents the first candidate and the second candidate according to the degree to which the plurality of antibodies contained in the cocktail antibody reagent match the plurality of antigens.

11. A reagent selection support method for supporting the selection of a cocktail antibody reagent containing a plurality of antibodies, the method comprising:
a step of acquiring a measurement order having a plurality of antigens as measurement targets;
a step of determining a plurality of the cocktail antibody reagents to be used in a plurality of assays for detecting a plurality of antigens based on the acquired measurement order; and
a step of outputting information of the plurality of cocktail antibody reagents to be used for the determined plurality of assays.

12. The reagent selection support method according to claim 11, further comprising:
a step of reading information of a plurality of cocktail antibody reagents configured by combinations of different antibodies from a storage unit;
wherein the step of determining a plurality of cocktail antibody reagents to be used in the plurality of assays is based on the measurement order and the information of the plurality of cocktail antibody reagents read from the storage unit in the reading step.

13. The reagent selection support method according to claim 12, wherein
each of the plurality of cocktail antibody reagents is configured by a combination of different antibodies.

14. The reagent selection support method of any one of claims 11 to 13, wherein
the determining step determines an antibody reagent that can be used in combination with the cocktail antibody reagent in at least one of the plurality of assays; and
the output step outputs information of the antibody reagent that can be used in combination with the cocktail antibody reagent.

15. The reagent selection support method of any one of claims 11 to 14, wherein
the determining step determines, based on the measurement order, a first candidate to be a combination of cocktail antibody reagents used in the plurality of assays, and a second candidate to be a combination of cocktail antibody reagents different from the first candidate; and
the output step outputs the first candidate and the second candidate determined in the determining step.
